# EUROPÄISCHE PATENTSCHRIFT

(11) **EP 1 565 164 B1**
(45) Veröffentlichungstag und Bekanntmachung des Hinweises auf die Patenterteilung: **24.10.2012**
(21) Anmeldenummer: 03788818.7
(22) Anmeldetag: 24.11.2003
(51) Int. Cl.: A61K 9/127

(54) **SELBST-FORMENDE PHOSPHOLIPID GELE**
SELF-FORMING PHOSPHOLIPIDIC GELS
GELS PHOSPHOLIPIDIQUES SE FORMANT D'EUX-MEMES

(30) Priorität: 26.11.2002 DE 10255285
(43) Veröffentlichungstag der Anmeldung: 24.08.2005
(73) Patentinhaber: MCS Micro Carrier Systems GmbH, 41460 Neuss (DE)
(72) Erfinder: DIEDERICHS, Julia, Eva, 40227 Düsseldorf (DE)
(74) Vertreter: Arth, Hans-Lothar
(86) Internationale Anmeldenummer: PCT/DE2003/003883
(87) Internationale Veröffentlichungsnummer: WO 2004/047790

(56) Entgegenhaltungen:
- WO-A-98/02184
- DE-A- 4 003 783
- US-A- 4 944 948
- US-A- 5 432 196

## Beschreibung

Die Erfindung betrifft selbst-formende Gele bestehend aus natürlichen, halb-synthetischen und synthetischen Phospholipiden und Wasser.

Die Gele können als solche zur feuchtigkeitsspendenden oder beruhigenden Behandlung der Haut, Schleimhaut, natürlicher oder operativ entstandener Körperhöhlungen genutzt werden, oder pharmakologisch aktive Substanzen enthalten, die auf oder in die Haut, Schleimhaut, natürliche oder operativ entstandene Körperhöhlungen oder -kompartimente freigesetzt werden. Die Gele können als Stabilisator oder Solubiliser pharmazeutischen Formulierungen zugesetzt werden.

Phospholipide werden in Form von Liposomen als topische Arzneisoffträger. *[*Schreier & Bouwstra, J. Control. Release 30, 1-15, 1994; Cevc, Crit. Rev. Ther. Drug Carrier Syst. 13, 257-288, 1996; Yarosh, Photodermatol. Photoimmunol. Photomed. 17, 203-212, 2001*]* und als Bestandteile von kosmetischen Zubereitungen wie Cremes und Lotionen *[*Weiner et al., J. Drug Target. 2, 405-410, 1994*]* eingesetzt. Gewöhnlich werden Liposome direkt in ihrer wäßrigen, dispersen Form verwendet oder in eine gel-bildende Matrix einschließlich pharmazeutisch verwendeter Basiscremes oder Hydrogele eingearbeitet.

Dennoch sind auch mehrere Arten von Phospholipid Gelen und ihr entsprechender Herstellungsprozesse beschrieben. Ghyczy und Mitarbeiter *[*Ghyczy et al., EP 0514435 B1*]* beschreiben ein alkoholisches Phospholipid Gel mit einem Phospholipidgehalt von 15-30% und 14-20% Alkohol. Dreidimensionale Liposomen Netzwerke aus hochkonzentrierten (60%) halbfesten Phospholipid Dispersionen sind von Brandl und Mitarbeitern entwickelt und charakterisiert worden. *[*Brandl et al., Adv. Drug Deliv. Rev. 24, 161-164, 1997; Brandl et al., Chem. Phys. Lipids 87, 65-72, 1997; Brandl et al. US 6,399,094*].* Vesikuläre Phospholipid Gele, die aus 40% Phosphatidylcholin und Cholesterol bestehen, sind als Träger für Zytostatika zur lokalen Behandlung von Krebs angewendet worden *[*Moog et al., J. Liposome Res. 8, 87-88, 1998; Güthlein et al., J. Liposome Res. 10, 251-252, 2000*;* Unger et al., WO 99149716*].* Ibscher [Dissertation, Universität Freiburg, Deutschland, 2000*;* Ibscher & Fridrich, WO 01/13887 A2*]* entwickelte ein Phospholipid Gel als topischer Träger zur antiviralen Behandlung der Haut bestehend aus Phospholipiden, Alkoholen und Zuckeralkoholen oder Kohlenhydraten. Vesikuläre Systeme bestehend aus einem geringen Phospholipid (2%) und einem hohen Alkohol Gehalt (30%), sogenannte Ethosomen, sind ebenfalls zur topischen Anwendung und zum Transport von aktiven Substanzen in die Haut beschrieben *[*Touitou et al., J. Control. Release 3, 403-418, 2000*;* Dayan & Touitou, Biomaterials 21, 1879-1885, 2000*;* Touitou, WO 95/35095*].*

Die US 4,944,948 offenbart hochviskose wässrige Dispersionen aus negativ geladenen Liposomen, in denen EGF eingeschlossen ist.

DE 40 03 783 offenbart Phospholipidgele, welche als liposomale Lösung eingesetzt werden können, wobei sich die Gele durch kräftiges Mischen der Phospholipide zusammen mit Alkohol und anschließender Zugabe von Wasser ausbilden.

Die US 5,432,196 offenbart ein Verfahren zur Herstellung von aktiven wässrigen Substanzen durch Mischung von Anipamil Hydrochlorid mit einem Phospholipid. Das Gemisch wird dann bei erhöhter Temperatur und durch Zugabe von Wasser kräftig gemischt, bis sich ein Gel formt. Durch die weitere Zugabe von Wasser bei erhöhter Temperatur, wird die Konzentration der aktiven Substanz im Gel angepasst.

WO 98/02184 offenbart eine pharmazeutische Zubereitung zur Therapie von durch Viren bedingten Erkrankungen der Haut. Die beschriebenen Zubereitungen enthalten Aciclovir, ein Phospholipid oder ein Phospholipid-Gemisch, Alkohol und Wasser und formen sich durch kräftiges mischen bei erhöhter Temperatur,

Im Gegensatz zu den obigen Systemen wurde überraschenderweise festgestellt, dass neutrale und negativ geladene Phospholipide in niedrigen Konzentrationen gemischt in Wasser spontan Gele bilden, die stabil genug sind, um weiter verarbeitet zu werden, z.B in Behältnisse oder Spritzen gefüllt, und auf die menschliche Haut oder Körper-Kompartimente appliziert zu werden. Darüber hinaus stabilisieren die Gele pharmazeutische Formulierungen, d.h. es werden schwerlösliche Stoffe in Lösung gehalten und Präzipitation verhindert.

Gegenstand der vorliegenden Erfindung ist ein Phospholipid-Gel bestehend aus einem neutralen und einem negativ geladenem Phospholipid und Wasser.

Die in der Gelen der vorliegenden Erfindung verwendeten Phospholipide können aus natürlichen, halbsynthetischen oder synthetischen Phospholipiden ausgewählt werden.

Die erfindungsgemäß verwendeten Phospholipide können aus natürlichen, halbsynthetischen und synthetischen Phospholipiden ausgewählt werden. Geeignete Phospholipide, die in dem erfindungsgemäßen Phospholipidgel eingesetzt werden können, sind z.B. Phosphatidylcholine. Beispiele für natürliche neutrale Phospholipide sind Soja- Phosphatidylcholin und Ei-Phosphatidylcholin. Als synthetische oder halb-synthetische Phospholipide können beliebige Fettalkanoylphosphatidylcholine, insbesondere solche die sich von gesättigten oder ungesättigten C₈-C₂₂-Alkanoylphosphatidylcholine ableiten, eingesetzt werden. Die Fettalkanoylreste leiten sich beispielsweise ab von der Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Tubercolostearinsäure, Palmitoleinsäure, Ölsäure, Erucasäure, Linolsäure, Linolensäure, Elaeostearinsäure, Arachidonsäure, Clupanodonsäure, Docosahexaensäure und deren beliebigen Gemischen ab. Ein bevorzugt eingesetztes Phosphatidylcholin ist Dipalmitoylphosphatidylcholin.

Als negativ geladene Phospholipide kommen insbesondere solche in Betracht, die einen Abonsäuresalz-rest im Molekül enthalten. Beispiele für negativ geladene Phospholipide sind zum Beispiel Phosphatidylglycerol, dass zu den natürlich vorkommenden negativ geladenen Phospholipiden zählt. Weitere Beispiele sind Dialkanoylphosphatidylglycerole, wobei der Alkanoylrest sich von den oben genannten Fettsäuren ableiten kann. Als Beispiele für geeignete Dialkanoylphosphatidylglycerole können Dipalmitoylphosphatidylglycerol und Dimyrrestoylphosphatidylglycerol genannt werden. Als negativ geladene Phospholipide sind auch Phosphatidylserin und Phosphatidsäure geeignet, die jeweils auch Fettsäureketten im Molekül enthalten können, wobei sich auch hier die Fettsäureketten von den oben genannten Fettsäuren ableiten können, wie beispielsweise von Palmitinsäure. Ein weiteres negativ geladenes Phospholipid ist z.B. Phosphatidylinositol. Die negativ geladenen Phospholipide weisen als kationisches Gegenion vorzugsweise Alkalionen oder Ammoniumionen auf. Die Auswahl des Kations ist nicht auf bestimmte Kationen beschränkt, sofern diese physiologisch verträglich sind.

In dem erfindungsgemäßen Phospholipidgel liegt die Gesamt-Phospholipidkonzentration vorzugsweise in einem Bereich zwischen 6 und 40 Gewichts-%. Das Verhältnis von neutralem Phospholipid zu negativ geladenem Phospholipid kann in weiten Bereichen ausgewählt werden, vorzugsweise liegt das Verhältnis von neutralem Phospholipid zu negativ geladenem Phospholipid im Bereich von 10:0,01 bis 10:5, insbesondere von 10:1 bis 10:0,25.

In das erfindungsgemäße Phospholipidgel können beliebige pharmakologisch aktive Substanzen eingearbeitet werden. Beispiele für die aktive Substanz sind Steroide, nicht-steroidale Antiflogistika, Antibiotika, Antioxidantien, oder Antiepileptika. Die Steroide können beispielsweise ausgewählt werden aus der Gruppe bestehend aus Hydrocortison oder Dexamathason, das nicht-steroidale Antiphlogistikum ausgewählt ist aus der Gruppe bestehend aus Ibuprofen, Diclofenac, Flurbiprofen oder Nabumeton, das Antibiotikum ausgewählt ist aus der Gruppe bestehend aus Tetracyclin oder eines seiner Derivate, einem Aminoglykosid wie z.B. Gentamycin oder Neomycin, einem Macrolid-Antibiotikum wie z.B. Erythromycin, einem Nitroimidazol Derivat wie z.B. Metronidazol oder Flucidinsäure, ein antibiotisches Peptid oder einem antibiotisches Oligonukleotid, das Antioxidans ausgewählt ist aus der Gruppe bestehend aus Vitamin E oder Coenzym Q₁₀, das Antiepileptikum ausgewählt ist aus der Gruppe bestehend aus Valproinsäure und ihren Salzen.

Als Beispiel für eine besonders geeignete Ausführungsform sei ein Gemisch aus Soja-Phosphatidylcholin und Phosphatidylglycerol genannt.

Neutrale Phospholipide in einer Konzentration von beispielsweise 5 bis 30%, insbesondere von 5 bis 20% und negativ geladene Phospholipide in einer Konzentration von beispielsweise 0,25 bis 10% formen, wenn sie mit Wasser gemischt werden, spontan ein Gel. Dies kann beispielsweise mit den natürlichen Komponenten Soja-Phosphatidylcholin und Phosphatidylglycerol erfolgen, aber auch mit Mischungen aus synthetischen Phosphatidylcholin/Phosphatiylglycerol wie Dipalmitoylphosphatidylcholin und Dipalmitoylphosphatidylglycerol oder Dimyristoylphosphatidylglycerol erreicht werden. Das Gel bildet sich spontan aus einem dünnen Lipidfilm, wenn dieser unter sanftem Schütteln in Wasser dispergiert wird.

Dispersion unter starker Scherung und hohem Druck (Hochdruckhomogenisation) ist nicht erforderlich. Organische Lösungsmittel, Detergentien oder Brücken-bildende zweiwertigen Ionen sind ebenfalls nicht notwendig. Aktive Substanzen können in das Gel eingearbeitet werden; insbesondere Substanzen vom Ubiquinone-Typ wie Coenzym Q₁₀ können vorhanden sein ohne die Ausbildung und Stabilität der Gelstruktur zu stören.

### Beispiele

### Beispiel 1

**1A:** 180 mg Soja-Phosphatidylcholin und 20 mg Ei-Phosphatidylglycerol werden als dünner Film an einer Glaswand abgeschieden. Es werden 1 ml destilliertes Wasser dazu gegeben und der Behälter auf einem Schüttler bei niedriger Geschwindigkeit geschüttelt bis sich ein Gel gebildet hat. Das Gel wird in eine Spritze überführt und bei 4°C gelagert.
**1 B:** Der selbe Vorgang wird durchgeführt um ein Gel aus 190 mg Phosphatidylcholin und 10 mg Phosphatidylglycerol zu formen.
**1C:** Der selbe Vorgang wird durchgeführt um ein Gel aus 195 mg Phosphatidylcholin und 5 mg Phosphatidylglycerol zu formen.
**1D:** Der selbe Vorgang wird durchgeführt um ein Gel aus 90 mg Phosphatidylcholin und 10 mg Phosphatidylglycerol zu formen.
**1E:** Der selbe Vorgang wird durchgeführt um ein Gel aus 360 mg Phosphatidylcholin und 40 mg Phosphatidylglycerol zu formen.

### Beispiel 2

**2A:** 150 mg Dipalmitoylphosphatidylcholin und 15 mg Dimyristoylphosphatidylglycerol werden als dünner Film an einer Glaswand abgeschieden. Es werden 1 ml destilliertes Wasser dazu gegeben und der Behälter auf einem Schüttler bei niedriger Geschwindigkeit geschüttelt bis sich ein Gel gebildet hat. Das Gel wird in eine Spritze überführt und bei 4°C gelagert
**2B:** Der selbe Vorgang wird durchgeführt um ein Gel aus 100 mg Dipalmitoylphosphatidylcholin und 10 mg Dimyristoylphosphatidylglycerol zu formen.
**2C:** Der selbe Vorgang wird durchgeführt um ein Gel aus 60 mg Dipalmitoylphosphatidylcholin und 6 mg Dimyristoylphosphatidylglycerol zu formen.

### Beispiel 3

180 mg Dipalmitoylphosphatidylcholin und 20 mg Dimyristoylphosphatidylglycerol werden mit 30 mg Coenzyme Q₁₀ in Chloroform vereinigt. Das organische Lösungsmittel wird unter Vakuum verdampft und die verbleibende Phospholipid-Q₁₀ Mischung als dünner Film an einer Glaswand abgeschieden. Es werden 1 ml destilliertes Wasser dazu gegeben und der Behälter auf einem Schüttler bei niedriger Geschwindigkeit geschüttelt bis sich ein Gel gebildet hat. Das Gel wird in eine Spritze überführt und bei 4°C gelagert.

## Patentansprüche

1. Phospholipidgel nur bestehend aus einem neutralen und einem negativ geladenen Phospholipid und Wasser, **dadurch gekennzeichnet, dass** sich das Gel spontan aus den Komponenten bildet.

2. Phospholipidgel nach Anspruch 1, worin die beiden Phospholipid Komponenten entweder natürlichen, halb-synthetischen oder synthetischen Ursprungs sind.

3. Phospholipidgel nach Anspruch 1 oder 2, worin die beiden Phospholipide ausgewählt sind aus Di(C₈-C₂₂-acyl)phosphatidylcholin, bevorzugt Dipalmitoylphosphatidylcholin, und Di(C₈-C₂₂-acyl)phosphatiylglycerol, bevorzugt Dipalmitoylphosphatidylcholin.

4. Phospholipidgel nach einem der Ansprüche 1 bis 3, worin Gesamtphospholipidkonzentration im Bereich von 6-40 Gewichtsprozent liegt.

5. Phospholipidgel nach einem der Ansprüche 1 bis 4, worin das Verhältnis von Phosphatidylcholin zu Phosphatidylglycerol im Bereich von 10:1 bis 10:0.25 liegt.

6. Phospholipidgel nur bestehend aus einem neutralen und einem negativ geladenen Phospholipid und Wasser, **dadurch gekennzeichnet, dass** sich das Gel spontan aus den Komponenten bildet, wobei zusätzlich eine pharmakologisch aktive Substanz enthalten ist und worin die pharmakologisch aktive Substanz ein Steroid, ein nicht-steroidales Antiphlogistikum, ein Antibiotikum, ein Antioxidants oder ein Antiepileptikum ist.

7. Phospholipidgel nach Anspruch 6, wo das Steroid ausgewählt ist aus der Gruppe bestehend aus Cholesterol, Hydrocortison oder Dexamathason, das nicht-steroidale Antiphlogistikum ausgewählt ist aus der Gruppe bestehend aus Ibuprofen, Diclofenac, Flurbiprofen oder Nabumeton, das Antibiotikum ausgewählt ist aus der Gruppe bestehend aus Tetracyclin oder eines seiner Derivate, einem Aminoglykosid wie z.B. Gentamycin oder Neomycin, einem Macrolid-Antibiotikum wie z.B. Erythromycin, einem Nitroimidazol Derivat wie z.B. Metronidazol oder Flucidinsäure, ein antibiotisches Peptid oder einem antibiotischen Oligonukleotid, das Antioxidans ausgewählt ist aus der Gruppe bestehend aus Vitamin E oder Coenzym Q₁₀, das Antiepileptikum ausgewählt ist aus der Gruppe bestehend aus Valproinsäure und ihren Salzen.

8. Phospholipidgel nach einem der Ansprüche 1 bis 7 entstanden durch jegliche nützliche Herstellungstechnik inklusive Schütteln, Vortexen, mischen mit einem Rührer, Extrusion und Homogenisation.

9. Verwendung des Phospholipidgels nach einem der Ansprüche 1 bis 8 als feuchtigkeits-spendende oder beruhigende Substanz auf normaler oder erkrankter Haut oder Schleimhaut.

10. Verwendung des Phospholipidgels nach einem der Ansprüche 1 bis 8 als Träger für Arzneistoffe oder kosmetische Substanzen zur Anwendung auf der Haut, Schleimhaut, in natürlichen Körperhöhlungen oder Körperkompartimenten erreichbar durch lokale Applikation.

11. Verwendung des Phospholipidgels nach einem der Ansprüche 1 bis 8 als Stabilisator zugesetzt zu einer Lösung zur Solubilisierung schwerlöslicher Substanzen und/oder zur Verhinderung von Präzipitationen.

## Claims

1. A phospholipid gel only consisting of a neutral phospholipid and a negatively charged phospholipid and water, **characterized in that** the gel forms itself spontaneously from the components.

2. The phospholipid gel according to claim 1, wherein both phospholipid components are either of natural origin, semi-synthetic origin, or synthetic origin.

3. The phospholipid gel according to claim 1 or 2, wherein the two phospholipids are selected from di(C₈-C₂₂-acyl)phosphatidyl choline, preferably dipalmitoyl phosphatidyl choline, and di(C₈-C₂₂-acyl)phosphatidyl glycerol, preferably dipalmitoyl phosphatidyl choline.

4. The phospholipid gel according to any of claims 1 to 3, wherein the total phospholipid concentration is within a range of 6-40% by weight.

5. The phospholipid gel according to any of claims 1 to 4, wherein the ratio of phosphatidyl choline to phosphatidyl glycerol is within a range of 10:1 to 10:0.25.

6. A phospholipid gel only consisting of a neutral phospholipid and a negatively charged phospholipid and water, **characterized in that** the gel forms itself spontaneously from the components, wherein further a pharmacologically active substance is contained and wherein the pharmacologically active substance is a steroid, a non-steroidal antiphlogistic agent, an antibiotic, an antioxidant, or an antiepileptic agent.

7. The phospholipid gel according to claim 6, wherein the steroid is selected from the group consisting of cholesterol, hydrocortisone, or dexamethasone, the non-steroidal antiphlogistic agent is selected from the group consisting of ibuprofen, diclofenac, flurbiprofen, or nabumetone, the antibiotic is selected from the group consisting of tetracycline or one of its derivatives, an aminoglycoside such as gentamycine or neomycine, a macrolid antibiotic such as erythromycine, a nitroimidazole derivative such as metronidazole or flucidic acid, an antibiotic peptide or an antibiotic oligonucleotide, the antioxidant is selected from the group consisting of vitamin E or coenzyme Q₁₀, the antiepileptic agent is selected from the group consisting of valproic acid and its salts.

8. The phospholipid gel according to any of claims 1 to 7, produced by any useful production technique including shaking, vortexing, mixing by a stirrer, extrusion, or homogenization.

9. Use of the phospholipid gel according to any of claims 1 to 8 as a moisturizing or calming substance on normal or diseased skin or mucosa.

10. Use of the phospholipid gel according to any of claims 1 to 8 as a carrier for pharmaceutical drugs or cosmetic substances for treating the skin, mucosa, in natural body cavities or body compartments accessible by local application.

11. Use of the phospholipid gel according to any of claims 1 to 8 as a stabilizer added to a solution for solubilizing hardly soluble substances and/or for preventing precipitation.

## Revendications

1. Un gel de phospholipide juste consistant d'un phospholipide neutre et d'un phospholipide négativement chargé et d'eau, **caractérisé en ce que** le gel se forme de ses composants spontanément.

2. Le gel de phospholipide selon la revendication 1, dans lequel les deux composants du phospholipide sont ou d'origine naturelle, semi-synthétique ou synthétique.

3. Le gel de phospholipide selon la revendication 1 ou 2, dans lequel les deux phospholipides sont choisis de di(acyle en C8-C22)phosphatidylcholine, de préférence dipalmitoylphosphatidylcholine, et di(acyle en C8-C22)phosphatidylglycérol, de préférence dipalmitoylphosphatidylcholine.

4. Le gel de phospholipide selon une des revendications 1 à 3, dans lequel la concentration totale du phospholipide est entre 6 à 40% du poids.

5. Le gel de phospholipide selon une des revendications 1 à 4, dans lequel la relation de phosphatidylcholine à phosphatidylglycérol est entre 10 :1 à 10:0,25.

6. Un gel de phospholipide juste consistant d'un phospholipide neutre et d'un phospholipide négativement chargé et d'eau, **caractérisé en ce que** le gel se forme de ses composants spontanément, et additionnel contenant une substance pharmacologiquement active, dans lequel la substance pharmacologiquement active est un stéroïde, un agent pharmaceutique non stéroïdien antiphlogistique, un antibiotique, un antioxidant ou un agent antiépileptique.

7. Le gel de phospholipide selon la revendication 6, dans lequel le stéroïde est choisi parmi le groupe consistant de cholestérol, hydrocortisone ou dexaméthasone, l'agent pharmaceutique non stéroïdien antiphlogistique est choisi parmi le groupe consistant d'ibuprofène, diclofénac, flurbiprofène ou nabumétone, l'antibiotique est choisi parmi le groupe consistant de tétracycline ou un de ses dérivés, un aminoglycoside tel que gentamicin ou néomycine, un antibiotique macrolide tel que érythromycine, un dérivé de nitroimidazole tel que métronidazole ou acide flucidique, un peptide antibiotique ou un oligonucléotide antibiotique, l'antioxidant est choisi parmi le groupe consistant de vitamine E ou coenzyme Q₁₀, l'agent antiépileptique est choisi parmi le groupe consistant d'acide valproïque et ses sels.

8. Le gel de phospholipide selon une des revendications 1 à 7, produit par quelconque technique appropriée incluant agiter, mélanger au vortex, mélanger par un agitateur, extrusion ou homogénéisation.

9. Le gel de phospholipide selon une des revendications 1 à 8 comme substance hydratante ou calmante sur le peau ou la muqueuse normal ou malade.

10. Le gel de phospholipide selon une des revendications 1 à 8 comme transporteur d'agents pharmaceutiques ou de substances cosmétiques pour traiter le peau, la muqueuse, en cavités corporelles naturelles ou en compartiments corporels accessibles par application locale.

11. Le gel de phospholipide selon une des revendications 1 à 8 comme stabilisateur ajouté à une solution pour solubiliser des substances peu solubles et/ou pour prévenir une précipitation.
